(19) Europäisches Patentamt
European Patent Office
Office européen des brevets

(11) Publication number : 0 276 920 B1

(12) EUROPEAN PATENT SPECIFICATION

(45) Date of publication of patent specification : 03.04.91 Bulletin 91/14

(51) Int. Cl.$^5$ : C07D 237/08, C07D 239/26, C07D 241/12, A01N 43/60, A01N 43/54, A01N 43/58

(21) Application number : 88300222.2

(22) Date of filing : 12.01.88

# (54) (2-Cyano-2-(phenyl or naphthyl)-2-substituted-ethyl) pyrazines, pyrimidines and pyridazines.

(30) Priority : 28.01.87 US 7688

(43) Date of publication of application : 03.08.88 Bulletin 88/31

(45) Publication of the grant of the patent : 03.04.91 Bulletin 91/14

(84) Designated Contracting States : AT BE CH DE ES FR GB GR IT LI LU NL SE

(56) References cited :
EP-A- 0 028 755
GB-A- 1 481 990
US-A- 4 366 165

(73) Proprietor : ROHM AND HAAS COMPANY
Independence Mall West
Philadelphia Pennsylvania 19105 (US)

(72) Inventor : Shaber, Steven Howard
44 Ash Stoker Lane
Horsham Pennsylvania 19044 (US)
Inventor : Sharma, Ashok Kumar
116 Horseshoe Lane
Horsham Pennsylvania 19044 (US)
Inventor : Reynolds, Charles Howard
39 Hunt Drive
Horsham Pennsylvania 19044 (US)

(74) Representative : Angell, David Whilton et al
ROHM AND HAAS (UK) LTD. European Operations Patent Department Lennig House
2 Mason's Avenue
Croydon CR9 3NB (GB)

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Jouve, 18, rue Saint-Denis, 75001 PARIS

## Description

This invention is concerned with certain (2-cyano-2-(phenyl or naphthyl)-2-substituted ethyl) pyrazines, pyrimidines and pyridazines which are novel compounds possessing fungicidal activity, fungicidal compositions containing these compounds and to methods of controlling phytopathogenic fungi using these compounds and compositions.

Several phenyl-pyridyl-alkylnitriles are known. For example, US-A-3,397,273 discloses 3-pyridyl-methane derivatives and their use for controlling phytopathogenic fungi. Herbicides which are 2-phenyl-4-cyano-4-(3-pyridyl)butyrate esters or acids are disclosed in US-A-4,224,052, US-A-4,313,754 and US-A-4,383,848. Additionally, phenyl-triazole-alkylnitriles, specifically 1- and 4-arylcyanoalkyl-1,2,4-triazoles disclosed in US-A-4,366,165, are known to have fungicidal activity. However, none of these references teaches the class of compounds of the present invention.

We have now found a new class of 2-arylethyl-2-cyano-heterocycles which has the formula (I)

```
            CN
            |
Het-CH2-C-Ar          (I)
            |
            R
```

wherein Het is a heterocyclic group selected from pyrazinyl, pyrimidinyl and pyridazinyl, preferably pyrazinyl or pyrimidinyl ; R is hydrogen, ($C_1$-$C_8$)alkyl, ($C_3$-$C_6$)cycloalkyl, ($C_3$-$C_6$)cycloalkyl($C_1$-$C_4$)alkyl, halo($C_3$-$C_8$)alkyl, ($C_3$-$C_6$)alkenyl, halo($C_3$-$C_6$)alkenyl, ($C_3$-$C_6$)alkynyl, ($C_1$-$C_5$)alkoxy, ($C_2$-$C_5$)alkenoxy, ($C_2$-$C_5$)alkynoxy, optionally substituted phenyl or naphthyl, optionally substituted phenoxy, optionally substituted phenyl($C_1$-$C_4$)alkyl or optionally substituted phenoxy($C_2$-$C_4$)alkyl wherein when R is phenyl, phenoxy, naphthyl, phenylalkyl or phenoxy alkyl, the phenyl portion may optionally be substituted with up to two substituents each independently selected from halogen, nitro, trihalomethyl, cyano, ($C_1$-$C_4$)alkyl, ($C_1$-$C_4$)alkoxy, alkoxyalkyl having up to a total of four carbon atoms, ($C_1$-$C_4$)alkylthio, ($C_1$-$C_4$)alkylsulfinyl and ($C_1$-$C_4$)alkylsulfonyl groups ; and Ar is a phenyl or naphthyl group wherein the phenyl is optionally substituted with up to three, preferably up to two substituents, and wherein the naphthyl is optionally substituted with up to two and preferably one substituent. Preferred optional substituents on the phenyl and naphthyl groups of R and Ar are each independently selected from halogen, ($C_1$-$C_4$)alkyl, ($C_1$-$C_4$)alkoxy, nitro, trihalomethyl, ($C_1$-$C_4$)alkylthio (-Salkyl) and phenyl which may be substituted with up to one halogen. Acid salts, free bases and metal salt complexes of the compounds of formula I are also encompassed within the invention.

By haloalkyl or haloalkenyl we mean haloalkyl or haloalkenyl having up to nine halogen atoms, preferably no more than four halogen atoms and more preferably no more than three halogen atoms. Moreover, it is preferred that the halogenation of the alkyl or alkenyl occur near the terminal end of the substituent. Fluorine is the preferred halogen atom when R is haloalkyl and fluorine or chlorine are preferred when R is haloalkenyl. When the haloalkyl or halomethyl substituent is a substituent of the Ar moiety, then the substituents contains no more than three halogen atoms, preferably the halogenation occurs near the terminal end of the haloalkyl and fluorine is the preferred halogen ; trifluoromethyl is the more preferred haloalkyl or trihalomethyl.

The term alkyl as used herein to describe alkyl, haloalkyl, phenylalkyl or phenoxyalkyl includes both straight chained and branched alkyl groups. The term alkoxyalkyl also includes cyclic alkoxyalkyl groups, for example, tetrahydrofuryl.

When R is a branched alkyl, it is preferred that the branching not occur at the first carbon of the alkyl, i.e., the carbon attached to the ethyl chain of the (2-arylethyl-2-cyano)heterocycles, when R is ($C_1$-$C_4$)alkyl or at the first or second carbon of the alkyl when R is ($C_5$-$C_8$)alkyl.

Typical compounds encompassed by the present invention include :

1. 2-(4-chlorophenyl)-2-cyano-1-pyrazinylhexane
2. 2-(4-chlorophenyl)-2-cyano-1-(5-pyrimidinyl)hexane
3. 2-(4-chlorophenyl)-2-cyano-1-(3-pyridazinyl)hexane
4. 2-(4-chlorophenyl)-2-cyano-1-pyrazinyl-(6,6,6-trifluoro)hexane
5. 2-(4-chlorophenyl)-2-cyano-1-(4-pyrimidinyl)-(6,6,6-trifluoro)hexane
6. 2-(4-chlorophenyl)-2-cyano-1-(3-pyridazinyl)-(6,6,6-trifluoro)hexane
7. 2-cyano-2-(4-phenylphenyl)-1-pyrazinylhexane
8. 2-cyano-2-(4-phenylphenyl)-1-(5-pyrimidinyl)hexane
9. 2-cyano-2-(1-naphthyl)-1-pyrazinylhexane
10. 2-cyano-2-(1-naphthyl)-1-(5-pyrimidinyl)hexane

11. 2-cyano-2-(4-fluorophenyl)-1-pyrazinylpentane
12. 2-cyano-2-(4-fluorophenyl)-1-(5-pyrimidinyl)pentane
13. 2-cyano-2-(4-fluorophenyl)-1-(3-pyridazinyl)pentane
14. 2-chloro-4-cyano-4-(4-fluorophenyl)-5-pyrazinyl-1-pentene
15. 2-chloro-4-cyano-4-(4-fluorophenyl)-5-(4-pyrimidinyl)-1-pentene
16. 2-cyano-2-(4-fluorophenyl)-5-methyl-1-pyrazinylhexane
17. 2-cyano-2-(4-fluorophenyl)-5-methyl-1-(5-pyrimidinyl)hexane
18. 2-cyano-2-(4-fluorophenyl)-5-methyl-1-(3-pyridazinyl)hexane
19. 2-cyano-2-(2-methoxyphenyl)-1-pyrazinylpentane
20. 2-cyano-2-(2-methoxyphenyl)-1-(5-pyrimidinyl)pentane
21. 2-cyano-2-(2-methoxyphenyl)-1-(3-pyridazinyl)pentane
22. 2-cyano-2-(2-ethoxyphenyl)-1-pyrazinylpentane
23. 2-cyano-2-(2-ethoxyphenyl)-1-(4-pyrimidinyl)pentane
24. 2-cyano-2-phenyl-1-pyrazinylbutane
25. 2-cyano-2-phenyl-1-(5-pyrimidinyl)butane
26. 2-cyano-2-phenyl-1-(3-pyridazinyl)butane
27. 2-cyano-(1-naphthyl)-1-pyrazinylhexane
28. 3-(4-chlorophenyl)-2-cyano-2-phenyl-1-pyrazinylpropane
29. 3-(4-chlorophenyl)-2-cyano-2-phenyl-1-(5-pyrimidinyl)propane
30. 2-(4-chlorophenyl)-2-cyano-3-phenyl-1-pyrazinylpropane
31. 2-(4-chlorophenyl)-2-cyano-3-phenyl-1-(5-pyrimidinyl)propane
32. 2-cyano-2,3-di(4-chlorophenyl)-1-pyrazinylpropane
33. 2-cyano-2,3-di(4-chlorophenyl)-1-(5-pyrimidinyl)propane
34. 3-(3-chlorophenyl)-2-cyano-2-(4-fluorophenyl)-1-pyrazinylpropane
35. 3-(3-chlorophenyl)-2-cyano-2-(4-fluorophenyl)-1-(5-pyrimidinyl)propane
36. 3-(4-chlorophenyl)-2-cyano-1-pyrazinyl-2-(4-trifluoromethylphenyl)propane
37. 3-(4-chlorophenyl)-2-cyano-1-pyrazinyl-2-(4-trifluoromethyl)propane
38. 2-cyano-2,4-diphenyl-1-pyrazinylbutane
39. 2-cyano-2,4-diphenyl-1-(5-pyrimidinyl)butane
40. 4-(4-chlorophenyl)-2-cyano-2-phenyl-1-pyrazinylbutane
41. 4-(4-chlorophenyl)-2-cyano-2-phenyl-1-(5-pyrimidinyl)butane
42. 2-cyano-2-phenyl-4-trifluoromethylphenyl-1-pyrazinylbutane
43. 2-cyano-2-phenyl-4-trifluoromethylphenyl-1-(5-pyrimidinyl)butane
44. 2-cyano-2,4-di-(4-chlorophenyl)-1-pyrazinylbutane
45. 2-cyano-2,4-di-(4-chlorophenyl)-1-(5-pyrimidinyl)butane
46. 2-(2-chlorophenyl)-4-(4-chlorophenyl)-2-cyano-1-pyrazinylbutane
47. 2-(2-chlorophenyl)-4-(4-chlorophenyl)-2-cyano-1-(5-pyrimidinyl)butane
48. 2-(2-chlorophenyl)-2-cyano-4-(3-trifluoromethylphenyl)-1-pyrazinylbutane
49. 2-(2-chlorophenyl)-2-cyano-4-(3-trifluoromethylphenyl)-1-(5-pyrimidinyl)butane
50. 4-(4-chlorophenyl)-2-cyano-2-(3-fluorophenyl)-1-pyrazinylbutane
51. 4-(4-chlorophenyl)-2-cyano-2-(3-fluorophenyl)-1-(5-pyrimidinyl)butane
52. 4-(4-chlorophenyl)-2-cyano-2-(3-trifluoromethylphenyl)-1-pyrazinylbutane
53. 4-(4-chlorophenyl)-2-cyano-2-(3-trifluoromethylphenyl)-1-(5-pyrimidinyl)butane

The structures of above Compounds 1-53 are set forth in Table 1 below :

TABLE 1

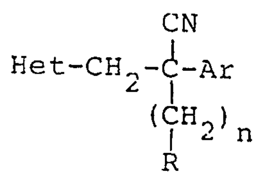

| Example | Het | Ar | R | n |
|---|---|---|---|---|
| 1. | A | Ø(4Cl) | $-(CH_2)_3CH_3$ | 0 |
| 2. | B | Ø(4Cl) | $-(CH_2)_3CH_3$ | 0 |
| 3. | C | Ø(4Cl) | $-(CH_2)_3CH_3$ | 0 |
| 4. | A | Ø(4Cl) | $-(CH_2)_3CF_3$ | 0 |
| 5. | D | Ø(4Cl) | $-(CH_2)_3CF_3$ | 0 |
| 6. | C | Ø(4Cl) | $-(CH_2)_3CF_3$ | 0 |
| 7. | A | Ø(4Ø) | $-(CH_2)_3CH_3$ | 0 |
| 8. | B | Ø(4Ø) | $-(CH_2)_3CH_3$ | 0 |
| 9. | A | 1-ØØ | $-(CH_2)_3CH_3$ | 0 |
| 10. | B | 1-ØØ | $-(CH_2)_3CH_3$ | 0 |
| 11. | A | Ø(4F) | $-(CH_2)_2CH_3$ | 0 |
| 12. | B | Ø(4F) | $-(CH_2)_2CH_3$ | 0 |
| 13. | C | Ø(4F) | $-(CH_2)_2CH_3$ | 0 |
| 14. | A | Ø(4F) | $-CH_2C(Cl)=CH_2$ | 0 |
| 15. | D | Ø(4F) | $-CH_2C(Cl)=CH_2$ | 0 |
| 16. | A | Ø(4F) | $-(CH_2)_2CH(CH_3)_2$ | 0 |
| 17. | B | Ø(4F) | $-(CH_2)_2CH(CH_3)_2$ | 0 |
| 18. | C | Ø(4F) | $-(CH_2)_2CH(CH_3)_2$ | 0 |
| 19. | A | Ø(2OCH$_3$) | $-(CH_2)_2CH_3$ | 0 |
| 20. | B | Ø(2OCH$_3$) | $-(CH_2)_2CH_3$ | 0 |
| 21. | C | Ø(2OCH$_3$) | $-(CH_2)_2CH_3$ | 0 |
| 22. | A | Ø(2OCH$_2$CH$_3$) | $-(CH_2)_2CH_3$ | 0 |
| 23. | D | Ø(2OCH$_2$CH$_3$) | $-(CH_2)_2CH_3$ | 0 |
| 24. | A | Ø | $-CH_2CH_3$ | 0 |
| 25. | B | Ø | $-CH_2CH_3$ | 0 |

| | | | | |
|---|---|---|---|---|
| 26. | C | Ø | $-CH_2CH_3$ | 0 |
| 27. | A | 1-ØØ | $-(CH_2)_3CH_3$ | 0 |
| 28. | A | Ø | Ø(4Cl) | 1 |
| 29. | B | Ø | Ø(4Cl) | 1 |
| 30. | A | Ø(4Cl) | Ø | 1 |
| 31. | B | Ø(4Cl) | Ø | 1 |
| 32. | A | Ø(4Cl) | Ø(4Cl) | 1 |
| 33. | B | Ø(4Cl) | Ø(4Cl) | 1 |
| 34. | A | Ø(4F) | Ø(3Cl) | 1 |
| 35. | B | Ø(4F) | Ø(3Cl) | 1 |
| 36. | A | Ø($4CF_3$) | Ø(4Cl) | 1 |
| 37. | B | Ø($4CF_3$) | Ø(4Cl) | 1 |
| 38. | A | Ø | Ø | 2 |
| 39. | B | Ø | Ø | 2 |
| 40. | A | Ø | Ø(4Cl) | 2 |
| 41. | B | Ø | Ø(4Cl) | 2 |
| 42. | A | Ø | Ø($4CF_3$) | 2 |
| 43. | B | Ø | Ø($4CF_3$) | 2 |
| 44. | A | Ø(4Cl) | Ø(4Cl) | 2 |
| 45. | B | Ø(4Cl) | Ø(4Cl) | 2 |
| 46. | A | Ø(2Cl) | Ø(4Cl) | 2 |
| 47. | B | Ø(2Cl) | Ø(4Cl) | 2 |
| 48. | A | Ø(2Cl) | Ø($3CF_3$) | 2 |
| 49. | B | Ø(2Cl) | Ø($3CF_3$) | 2 |
| 50. | A | Ø(3F) | Ø(4Cl) | 2 |
| 51. | B | Ø(3F) | Ø(4Cl) | 2 |
| 52. | A | Ø($3CF_3$) | Ø(4Cl) | 2 |
| 53. | B | Ø($3CF_3$) | Ø(4Cl) | 2 |

where

A = Pyrazinyl
B = 5-Pyrimidinyl
C = 3-Pyridazinyl
D = 4-Pyrimidinyl
Ø = Phenyl
ØØ = Naphthyl

The cyano-(phenyl or naphthyl)-ethyl heterocycles of the present invention can be prepared by conventional synthetic routes. For example, they may be prepares as shown by the following reaction Scheme (A) :

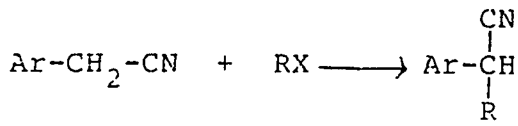

(1) (2) (3)

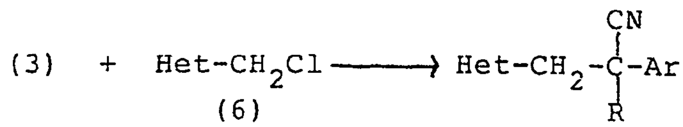

(6)

(I)

wherein R and Ar are as described previously for Formula (I) except that R is not a phenyl or heterocyclic group and X is a chloride, bromide, methylsulfonate, 4-tolylsulfonate, iodide, benzene sulfonate or another leaving group capable of effecting the desired reaction.

In reaction scheme (A) appropriately substituted arylcyanides (1) may be reacted with an organic halide, RX, under basic conditions at a temperature of from about − 20°C to about 50°C, preferably from about − 10°C to about 10°C. Examples of suitable bases include an alkali metal (preferably sodium or potassium) hydroxide and hydride, t-butoxide and dimsyl. Generally the hydroxide bases are used under phase transfer conditions in solvents, such as, methylene chloride, chloroform, carbon tetrachloride, benzene, toluene, ethers, tetrahydrofuran (THF) and dioxane. Hydride, t-butoxide and dimsyl bases are used in solvents, for example, toluene, dimethylsulfoxide (DMSO), dimethylformamide (DMF), glyme, ether and THF. The phase transfer conditions usually require catalysts. Suitable catalysts include tetrabutylammonium hydroxide, benzyltriethylammonium chloride or other quaternary ammonium salts, quaternary phosphonium salts and crown ethers, e.g., 18-crown-6. The resulting 2-substituted-2-phenylacetonitrile (3) is preferably purified, e.g., by distillation, and then reacted under basic conditions as described above at a temperature of from about 0°C to about 50°C with a halomethylheterocycle (6), e.g., chloromethylpyrazine, 5-(chloromethyl)pyrimidine or chloromethylpyridazine. The latter being prepared by the chlorination of a methylheterocycle, for example, with N-chlorosuccinimide (NCS). Chloromethylpyrazine can be prepared from methylpyrazine according to the procedures set forth in J. Org. Chem. Vol 38 (11) 2049-2052 (1973), Eur. J. Med. Chem. Ther. Vol 18 (6) 515-519 (1983) and J. Org. Chem. Vol 26 2356-2360 (1961). The chlorination of 5-methylpyrimidine to 5-(chloromethyl)pyrimidine is disclosed in Angew. Chem. Int. Ed. Vol 5 (7) 671 (1971). The product, a compound of Formula I, may be recovered from the reaction mixture as a free base or as a salt by conventional methods, e.g., adding an appropriate acid to precipitate the desired salt.

If only one reaction vessel is used, then it is preferred that at least three or four equivalents of base to benzyl cyanide (1) are used, that after the organic halide (2) is added, the reaction is allowed to proceed until essentially all of the alkylhalide is consumed before adding the halomethylheterocycle.

When R is a phenylalkyl group as previously described for Formula (I), then the appropriately substituted arylcyanide (1) may be reacted with RX where X is a methylsulfonate or a 4-tolylsulfonate under basic conditions created, for example, by sodium or potassium hydride in a solvent such as ether, dioxane, THF, toluene, DMF or DMSO at a temperature of from about − 20 to about 50° to obtain a 2-phenyl-2-phenylalkylacetonitrile (3). The desired cyano-aryl-ethyl-heterocycle (I) can then be obtained as described in Reaction Scheme A above. The phenylalkyl methylsulfonate or 4-tolylsulfonate can be obtained as shown by the following Reaction Scheme B :

$$\underset{(5)}{R\text{-}COOH} \longrightarrow \underset{(6)}{R\text{-}CH_2OH} \longrightarrow \underset{(2)}{R\text{-}X}$$

wherein R is phenylalkyl as described for Formula (I). The organic acid (5) is reduced, for example, with diborane or with lithium aluminum hydride in a solvent such as ether, THF or dioxane to obtain its corresponding alcohol (6). The alcohol (6) is reacted with methanesulfonyl chloride or 4-toluenesulfonyl chloride in the presence of an organic base such as pyridine or triethylamine in a solvent, for example, ether, methylene chloride or chloroform at at temperature of from about − 30 to about 10°C.

When R is a cycloalkyl or phenyl group, the compounds may be prepared as shown by following Reaction Scheme C :

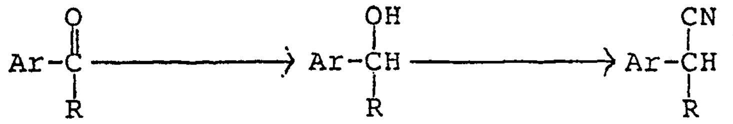

(7) (8) (3)

(C)

wherein R is a cycloalkyl or phenyl group as described previously for Formula (I) and Ar is as described previously for Formula (I).

An appropriately substituted arylketone (7) is reduced to its alcohol (8), for example, by reacting the ketone with sodium borohydride in methanol at reflux or with lithium aluminum hydride in ether at a temperature of from about −20 to about 30°C. The alcohol (8) is then converted to its methylsulfonate or 4-tolylsulfonate, for example, using the process described in Scheme B above. The resulting methylsulfonate or 4-tolylsulfonate is then reacted with sodium or potassium cyanide in a solvent, for example, acetonitrile, DMF or DMSO at a temperature of from about 30 to about 100°C to obtain a 2-substituted-2-phenylacetonitrile (3). The heterocycle (I) can then be obtained from this nitrile (3) as previously described in Scheme A.

Alternatively, when Ar and R are as described previously for Formula (I) except R is not a phenyl, the cyano-aryl-ethyl heterocycles can be prepared as shown in following Reaction Scheme D.

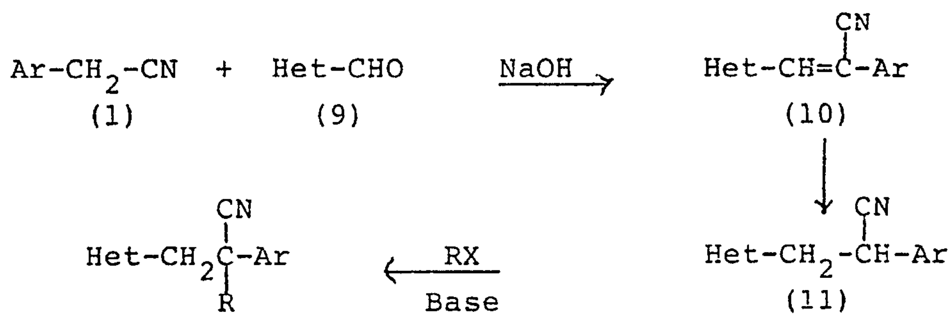


(1) (9) (10) (11) (I)

(D)

According to Reaction Scheme D, a suitably substituted arylcyanide (1) is reacted with a heterocyclic carboxaldehyde (9) under basic conditions to obtain an intermediate (10) which is subsequently reduced with potassium, lithium or, preferably, sodium borohydride to produce 2-cyano-2-aryl-heteroethane (11). The heteroethane (10) is alkylated with RX (2) under basic conditions to obtain a product of Formula (I).

The condensation of the arylcyanide (1) with the heterocyclic carboxaldehyde (9) may be conducted in a solvent, for example, an alcohol, ether, DMSO, DMF, toluene, a mixture thereof or water with one or more of these solvents, in the presence of a base at a temperature of from about −10°C to about 80°C. Preferably, the reaction is performed in an alcohol, i.e., methanol or ethanol, ether or toluene using a catalytic amount of an aqueous base, for example, sodium or potassium hydroxide, at a temperature of from about 0°C to about 20°C. The intermediate product (10) is reduced with potassium, or preferably, sodium borohydride in a solvent such as an alcohol, ether or DMF at a temperature of from about 0°C to about 50°C. Preferably, the reaction is carried out in methanol and at a temperature of from about 5°C to about 20°C.

The arylcyanides (1), organic halides (2), organic acids (5), organic ketones (7), and pyrazinecarboxaldehydes (9) can be obtained commercially or prepared by known methods.

The acid salts or metal salt complexes of the (2-cyano-2-(phenyl or naphthyl)-2-substituted-ethyl)heterocycles of this invention can be prepared by standard techniques known in the art. For example, the heterocycle of formula (I) can be dissolved in an appropriate solvent such as diethylether, tetrahydrofuran, ethanol, methanol, ethylacetate, hexane and toluene or combinations thereof and treated with an equivalent or excess amount of a mineral or organic acid which may or may not be dissolved in an appropriate solvent. The mixture is either cooled or evaporated to get the salt which can either be used as such or recrystallized from an appropriate solvent or combination of appropriate solvents.

The following Examples illustrate this invention.

Example 1 :

Preparation of 2-(4-Chlorophenyl)-2-Cyano-1-Pyrazinylhexane-Compound 1

A 2 liter flask fitted with a themometer, condenser and drying tube was charged 20.0 grams of methylpyrazine (1.0 eq., 0.21 moles), 36.8 grams of N-chlorosuccinimide (1.3 eq., 0.28 moles), 0.13 grams of benzoyl peroxide (0.25% eq.) catalyst and 600 ml of carbon tetrachloride. The mixture was refluxed at 80°C with stirring for 18 hrs. at which time solid was filtered off in vacuo and washed with 100 ml of carbon tetrachloride. The filtrate was dried over $MgSO_4$ and concentrated to give 22.0 grams of oil (80.9% yield). Purity of the crude product, from proton NMR integration, was 58% chloromethylpyrazine and 42% methylpyrazine (starting material).

Into a 200 ml round bottom flask under nitrogen was added 1.93 grams of 60% sodium hydride (2.0 eq., 0.048 moles). The sodium hydride was washed twice wich 25 ml of hexanes and flushed with 50 ml of DMF. Five grams of 2-(4-chlorophenyl)hexanenitrile (1.0 eq., 0.024 moles) in 25 ml of DMF was added dropwise to the sodium hydride. Evolution of hydrogen was observed and the suspension was stirred for 1 hr. Five grams of 2-chloromethylpyrazine (1.16 eq., 0.028 moles, 60% purity) in 25 ml of DMF was added to the reaction mixture while maintaining the temperature between 10-20°C followed by warming to room temperature. After stirring for 1 hr. at room temperature the reaction was determined complete by gas-liquid chromatography. The reaction was quenched by the addition of 100 ml of 10% HCl and extracted with 250 ml of ethyl acetate. The extract was washed twice with 100 ml of water, dried and concentrated to give 7.0 grams of a dark oil. The crude product was purified by flash chromatography and gave 3.4 grams of product (47.3% based on nitrile) as a light orange oil.

Examples 2 to 8

The compounds indicated below, and numbered according to Table 1, were prepared in a similar manner; the analytical data for the compounds being set forth in Table 2 below.

TABLE 2

| Compound | Formula | Elemental Analysis, Calculated (Found) | NMR (in $CDCl_3$) (90 MHz) | Melting Point (°C) |
|---|---|---|---|---|
| 1 | $C_{17}H_{18}N_3Cl$ | C = 68.11 (68.14)<br>H = 6.01 (6.08)<br>N = 14.02 (14.23)<br>Cl = 11.85 (11.51) | 0.7-1.0(m, 3H),<br>1.0-1.5(m, 4H),<br>2.0-2.2(m, 2H),<br>3.3-3.5(d, 2H),<br>7.0-7.6(m, 4H) and<br>8.2-8.5(m, 3H) | Oil |
| 2. | $C_{17}H_{18}N_3Cl$ | C = 68.11 (66.28)<br>H = 6.01 (5.92)<br>N = 14.02 (13.51)<br>Cl = 11.85 (13.79) | 0.7-1.0(m, 3H),<br>1.0-1.7(m, 4H),<br>2.0-2.3(m, 2H),<br>3.0-3.3(m, 2H),<br>7.0-7.7(m, 4H),<br>8.3 (s, 2H) and<br>9.1 (s, 1H) | Oil |
| 4. | $C_{17}H_{15}N_3ClF_3$ | C = 57.70 (57.49)<br>H = 4.74 (4.37)<br>N = 11.88 (12.43)<br>Cl = 10.04 (10.07)<br>F = 16.12 (15.88) | 1.2-2.4(m, 6H),<br>3.3-3.6(m, 2H),<br>7.0-7.6(m, 4H),<br>8.3(s, 1H) and<br>8.4-8.6(m, 2H) | 67-69°C |
| 7. | $C_{23}H_{23}N_3$ | C = 80.93 (79.10)<br>H = 6.74 (7.02)<br>N = 12.32 (12.14) | 0.7-1.0(m, 3H),<br>1.0-1.6(m, 4H),<br>2.0-2.3(m, 2H),<br>3.2-3.6(m, 2H),<br>7.1-7.7(m, 9H) and<br>8.2-8.6(m, 3H) | Oil |

| | | | | |
|---|---|---|---|---|
| 40. | $C_{21}H_{18}N_3Cl$ | C = 72.50 (72.30)<br>H = 5.18 (5.44)<br>N = 12.09 (11.70)<br>Cl = 10.20 (10.25) | 2.3-3.0(m, 4H),<br>3.3-3.6(m, 2H),<br>7.0-7.8(m, 9H),<br>8.2(d, 1H) and<br>8.4-8.6(m, 2H) | 65-67°C |
| 42. | $C_{22}H_{18}N_3F_3$ | C = 69.29 (69.38)<br>H = 4.72 (4.87)<br>N = 11.02 (10.98)<br>F = 14.96 (14.81) | 2.4-2.8(m, 4H),<br>3.5(s, 2H),<br>7.0-7.7(m, 9H),<br>8.2(d, 1H) and<br>8.3-8.5(m, 2H) | 97-99°C |
| 43. | $C_{22}H_{18}N_3F_3$ | C = 69.29 (69.34)<br>H = 4.72 (4.81)<br>N = 11.02 (10.99)<br>F = 14.96 (14.82) | 2.2-3.0(m, 4H),<br>3.0-3.9(m, 2H),<br>7.1-7.8(m, 9H),<br>8.3(s, 2H) and<br>9.1(s, 1H) | 92-94°C |
| 44. | $C_{21}H_{18}N_3Cl_2$ | C = 66.00 (65.83)<br>H = 4.45 (4.49)<br>N = 10.99 (10.88)<br>Cl = 18.59 (18.60) | 2.3-2.9(m, 4H),<br>3.3-3.6(m, 2H),<br>6.9-7.6(m, 8H),<br>8.3(d, 1H) and<br>8.4-8.6(m, 2H) | 106-110°C |

The compounds of this invention are useful in the preventative and curative treatment of phytopathogenic fungi, i.e., they may usefully be applied either before or after the plant's exposure to a fungus. They are effective against a broad spectrum of fungi, including those of the phycomycetes, ascomycetes, basidiomycetes and deuteromycetes classes. They are particularly effective against powdery mildews and rice blast. Consequently, various compounds of this invention may be useful in treating fungi which may affect cereal crops, fruit crops and vegetable crops.

The compounds of the invention can be applied as fungicidal sprays by methods commonly employed, such as conventional high-gallonage hydraulic sprays, low-gallonage sprays, air-blast, aerial sprays and dusts. The dilution and rate of application will depend upon the type of equipment employed, the method and frequency of application desired and diseases to be controlled, but the effective amount for application is usually from about 5 grams (gm) to about 22 kilograms (kg), preferably from about 0.010 to about 1.0 kg per hectare.

As a seed protectant, the amount of fungicide coated on the seed is usually at a dosage rate of about 0.0001 to about 10 grams (gm) and preferably from about 0.1 to about 10 gm per 1 kilogram of seed. As a soil fungicide the chemical can be incorporated in the soil or applied to the surface usually at a rate of 0.01 to about 22 kg, preferably about 0.05 to about 11 kg and more preferably from about 0.1 to about 3.3 kg per hectare. As a foliar fungicide the chemical can be applied at a rate of from about 0.01 to about 11 kg, preferably from about 0.02 to about 5.5 kg and more preferably from about 0.1 to about 3.3 kg per hectare.

The present invention is useful for the control of fungi and can be utilized at various loci such as the seed, the soil or the foliage. For such purposes these compounds can be used in the technical or pure form as prepared, as solutions or as formulations. The compounds are usually taken up in a carrier or are formulated so as to render them suitable for subsequent dissemination as fungicides. For example, these chemical agents can be formulated as wettable powders, emulsifiable concentrates, dusts, granular formulations, aerosols, or flowable emulsion concentrates. In such formulations, the compounds are extended with a liquid or solid carrier and, when desired, suitable surfactants are incorporated.

It is usually desirable, particularly in the case of foliar spray formulations, to include adjuvants, such as wetting agents, spreading agents, dispersing agents, stickers, adhesives and the like in accordance with agricultural practices. Such adjuvants commonly used in the art can be found in McCutcheon's Emulsifiers and Detergents, McCutcheon's Emulsifiers and Detergents/Functional Materials and McCutcheon's Functional Materials all published annually by McCutcheon Division of MC Publishing Company (New Jersey).

In general, the compounds of this invention can be dissolved in appropriate solvents such as acetone, methanol, ethanol, dimethylformamide or dimethyl sulfoxide and such solutions extended with water. The concentrations of the solution can vary from 1% to 90% with a preferred range being 5 to 50% (weight percentage).

For the preparation of emulsifiable concentrates, the compounds used in the invention can be dissolved in suitable organic solvents or a mixture of solvents, together with an emulsifying agent which permits dispersion of the fungicide in water. The concentration of the active ingredient in emulsifiable concentrates is usually 10% to 90% and in flowable emulsion concentrates, this can be as high as 75% (weight percent).

Water based flowable formulations of the compounds can be prepared with a concentration of active ingredients in the range of 5 to 70% by weight, preferably 20 to 50% by weight. A typical flowable formulation is prepared by wet-milling a mixture of 35 parts of 2-(4-chlorophenyl)-2-cyano-1-(5-pyrimidinyl)hexane, 10 parts of Barden clay, 4 parts of sodium lignosulfonate, 1 part of an anionic wetting agent and 50 parts of water.

Wettable powders suitable for spraying can be prepared by admixing the compound with a finely divided solid, such as clays, inorganic silicates and carbonates, and silicas and incorporating wetting agents, sticking agents, and/or dispersing agents in such mixtures. The concentration of active ingredients in such formulations is usually in the range of 5% to 98%, preferably 40% to 75% (weight percent). A typical wettable powder is made by blending 50 parts of 2-(4-chlorophenyl)-2-cyano-1-(5-pyrimidinyl)hexane, 45 parts of a synthetic precipitated hydrated silicon dioxide sold as Hi-Sil, 1 part of an anionic naphthalenic sulfonate wetting agent and 4 parts of sodium lignosulfonate (Marasperse N-22). In another preparation of a kaolin type (Barden) clay is used in place of the Hi-Sil in the above wettable powder and in another such preparation 25% of the Hi-Sil is replaced with a synthetic sodium silico aluminate sold as Zeolex 7.

Dusts are prepared by mixing the amides and salts and complexes thereof with finely divided inert solids which can be organic or inorganic in nature. Materials useful for this purpose include botanical flours, silicas, silicates, carbonates, talc and clays. One convenient method of preparing a dust is to dilute a wettable powder with a finely divided carrier. Dust concentrates containing 20% to 80% (weight percent) of the active ingredient are commonly made and are subsequently diluted to 1% to 10% use concentration.

The compounds of the present invention may also be utilized in combination with other fungicides such as :

(a) dithiocarbamates and derivatives such as :
ferric dimethyldithiocarbamate (ferbam), zinc dimethyldithiocarbamate (ziram), manganese ethylenebisdithiocarbamate (maneb) and its coordination product with zinc ion (mancozeb), zinc ethylenebisdithiocarbamate (zineb), zinc propylenebisdithiocarbamate (propineb), sodium methyldithiocarbamate (metham), tetramethylthiuram disulfide (thiram), the complex of zineb and polyethylene thiuram disulfide, 3,5-dimethyl-1,3,5-2H-tetrahydrothiadiazine-2-thione (dazomet) ; and mixtures of these and mixtures with copper salts ;

(b) nitrophenol derivatives such as :
dinitro-(1-methylheptyl) phenyl crotonate (dinocap), 2-sec-butyl-4,6-dinitrophenyl-3,3-dimethylacrylate (binapacryl), and 2-sec-butyl-4,6-dinitrophenyl isopropyl carbonate ;

(c) heterocyclic structures such as :
Systhane (a registered trademark of Rohm and Haas for myclobutanil), triademifon, N-trichloromethylthiotetrahydrophthalimide (captan), N-trichloromethylthiophthalimide (folpet), 2-heptadecyl-2-imidazole acetate (glyodine), 2-octylisothiazolone-3, 2,4-dichloro-6-(o-chloroanilino)-s-triazine, diethyl phthalimidophosphorothioate, 4-butyl-1,2,4-triazole, 5-amino-1-[bis(dimethylamino)phosphinyl]-3-phenyl-1,2,4-triazole, 5-ethoxy-3-trichloromethyl-1,2,4-thiadiazole, 2,3-dicyano-1,4-dithiaanthraquinone (dithianon), 1,3-dithiolo-[4,5-b]quinoxaline-2-thione (thioquinox), methyl 1-(butylcarbamoyl)-2-benzimidazole carbamate (benomyl), 2-4'-(thiazolyl) benzimidazole (thiabendazole), 4-(2-chlorophenylhydrazono)-3-methyl-5-isoxazolone, 3-(3,5-dichlorophenyl)-5-ethenyl-5-methyl-2,4-oxazolidinedione (vinclozolin), 3-(3,5-dichlorophenyl)-N-(1-methylethyl)-2,4-di-oxo-1-imidazolinecarboxamide (iprodione), N-(3,5-dichlorophenyl)-1,2-dimethylcyclopropane-1,2-dicarboximide (procymidone), beta-(4-chlorophenoxy)-alpha-(1,1-dimethylethyl)-1H-1,2,4-triazole-1-ethanol (triadimenol), 1-(4-chlorophenoxy)-3,3-dimethyl-1-(1H-1,2,4-triazol-1-yl)-2-butanone (triadimefon), beta-[(1,1'-biphenyl)-4-yloxy]-alpha-(1,1-dimethylethyl)-1H-1,2,4-triazole-1-ethanol (bitertanol), 2,3-di-

chloro-N-(4-fluorophenyl)maleimide (fluoroimide), 1-[2-(2,4-dichlorophenyl)-4-propyl-1,3-dioxolan-2-ylmethyl]-1H-1,2,4-triazole, pyridine-2-thiol-1-oxide, 8-hydroxyquinoline sulfate and metal salts thereof, 2,3-dihydro-5-carboxanilido-6-methyl-1,4-oxathiin-4,4-dioxide,
2,3-dihydro-5-carboxanilido-6-methyl-1,4-oxathiin-alpha-(phenyl)-alpha-(2,14-dichlorophenyl)-5-pyrimidinylmethanol(triarimol), cis-N-[(1,1,2,2-tetrachloroethyl)thio]-4-cyclohexene-1,2-dicarboximide, 3-[2-(3,5-dimethyl-2-oxycyclohexyl)-2-hydroxy]-glutarimide (cycloheximide), dehydroacetic acid, N-(1,1,2,2-tetrachloroethylthio)-3a,4,7,7a-tetrahy drophthalimide (captafol), 5-butyl-2-ethylamino-4-hydroxy-6-methyl-pyrimidine (ethirimol), acetate of 4-cyclodecyl-2,6-dimethylmorpholine(dodemorph), and 6-methyl-2-oxo-1,3-dithiolo[4,5-b]-quinoxaline (quinomethionate).
(d) miscellaneous halogenated fungicides such as :
tetrachloro-p-benzoquinone (chloranil), 2-3-dichloro-1,4-naphthoquinone (dichlone), 1,4-dichloro-2,5-dimethoxybenzene (chlorneb), 3,5,6-trichloro-o-anisic acid (tricamba), 2,4,5,6-tetrachloroisophthalonitril (TCPN), 2,6-dichloro-4-nitroaniline (dichloran), 2-chloro-1-nitropropane, polychloronitrobenzenes such as: pentachloronitrobenzene (PCNB) and tetrafluorodichloroacetone ;
(e) fungicidal antibiotics such as :
griseofulvin, kasugamycin and streptomycin ;
(f) copper-based fungicides such as :
copper hydroxide, cuprous oxide, basic cupric chloride, basic copper carbonate, copper terphthalate, copper naphthenate and Bordeaux mixture ; and
(g) miscellaneous fungicides such as :
diphenyl, sultone, dodecylguanidine acetate (dodine), phenylmercuric acetate, N-ethyl-mercuri-1,2,3,6-tetrahydro-3,6-endomethan-o-3,4,5,6,7,7-hexachlorophthalimide, phenyl-mercuric monoethanol ammonium lactate, p-dimethylaminobenzene sodium sulfonate, methyl isothiocyanate, 1-thiocyano-2,4-dinitrobenzene, 1-phenylthiosemicarbazide, nickel-containing compounds, calcium cyanamide, lime sulfur, 1,2-bis(3-methoxycarbonyl-2-thioureido) benzene (thiophanate-methyl).

The compounds were tested (not necessarily at the same time) for their fungicidal activity in vivo against wheat powdery mildew (WPM) and rice blast (RB). The compounds were dissolved in a 1 : 1 mixture of acetone and methanol, sprayed onto the plants and allowed to dry for about 24 hours before inoculating with the fungus. Each test utilized control plants which were sprayed with the solvent mixture and inoculated with the fungus. The remainder of the technique of each of the tests is given below.

A. Wheat Powdery Mildew (WPM)

Erysiphe graminis (f. sp. tritici) was cultured on Pennol or Hart wheat seedlings in a controlled temperature room at 65° to 70°F. Mildew spores were shaken from the culture plants onto Pennol or Hart wheat seedlings which had been previously sprayed with the fungicide compound. The inoculated seedlings were kept in a controlled temperature room at 65° to 75° and subirrigated. The percent disease control was rated 8 to 10 days after the inoculation.

B. Rice Blast (RB)

Untrimmed Nato or cultivar M-201 rice plants were inoculated with Piricularia oryzae (about 20,000-30,000 conidia per ml) by spraying the leaves and stems until a uniform film of inoculum was observed on the leaves. The inoculated plants were incubated in a humid environment (75° to 85°F) for 24 or 48 hours, then placed in a greenhouse environment (70° to 75°F). Seven to eight days after inoculation, the percent disease control was determined.

The inoculum was produced on plates of oatmeal agar containing 50 gm of Gerber brand baby oatmeal, 20 gm of bacto agar, 10 gm bacto dextrose and 1000 ml deionized water. The plates were inoculated with mycelial plugs (7-14 days old) Piricularia oryzae and maintained at room temperature under constant fluorescent light for 10-14 days. The plates were then flooded with a solution of 0.25 gm sodium oleate, 2 gm gelatin and 1000 ml deionized water and the plates scraped to release condia. The resulting mixture was filtered through cheesecloth and the spore suspension adjusted using a hemacytometer.

The results of the tests against wheat powdery mildew are reported in Table 3 as percent control (percentages of plants treated with the compounds of the present invention lacking disease sign or symptoms compared to the untreated control plants).

TABLE 3

Wheat Powdery Mildew

| Compound (100 ppm) | Percent Disease Control |
|---|---|
| 1 | 100 |
| 2 | 99 |
| 4 | 100 |
| 7 | 95 |
| 40 | 100 |
| 42 | 85 |
| 43 | 85 |
| 44 | 75 |

Some of the compounds showed good control of rice blast as well as wheat powdery mildew. These results are reported in Table 4.

TABLE 4

Rice Blast

| Compound | Percent Disease Control |
|---|---|
| 2 (25 ppm) | 80 |
| 7 (25 ppm) | 80 |
| 43 (100 ppm) | 80 |

In its mechanical aspect the process of the invention comprises-Claim 9 from ESP/POR
The following words are trademarks which may be registered :
Hi-Sil, Marasperse, Zeolex, Gerber

## Claims

### Claims for the contracting States : AT, BE, CH, DE, FR, GB, GR, IT, LI, LU, NL, SE

1. The compounds of the formula

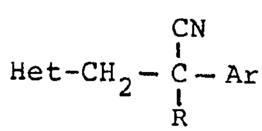

wherein Het is pyrazinyl, pyrimidinyl or pyridazinyl ;

R is hydrogen, $(C_1\text{-}C_8)$alkyl, $(C_3\text{-}C_6)$cycloalkyl, $(C_3\text{-}C_6)$cycloalkyl$(C_1\text{-}C_4)$alkyl, halo$(C_1\text{-}C_8)$alkyl, $(C_3\text{-}C_6)$alkenyl, halo$(C_3\text{-}C_6)$alkenyl, $(C_3\text{-}C_6)$alkynyl, $(C_1\text{-}C_5)$alkoxy, $(C_2\text{-}C_5)$alkenoxy, $(C_2\text{-}C_5)$alkynoxy, substituted or unsubstituted phenyl, substituted or unsubstituted naphthyl, substituted or unsubstituted phenoxy, substituted or unsubstituted phenyl $(C_1\text{-}C_4)$alkyl, or substituted or unsubstituted phenoxy $(C_2\text{-}C_4)$alkyl, wherein when R is a phenyl, phenoxy, naphthyl, phenylalkyl or phenoxy alkyl, the phenyl portion may optionally be substituted with up to two substituents each independently selected from halogen, nitro, trihalomethyl, cyano, $(C_1\text{-}C_4)$alkyl, $(C_1\text{-}C_4)$alkoxy, alkoxyalkyl having up to a total of four carbon atoms, $(C_1\text{-}C_4)$alkylthio,

($C_1$-$C_4$)alkylsulfinyl and ($C_1$-$C_4$)alkylsulfonyl groups ; and

Ar is a substituted or unsubstituted phenyl or naphthyl group, wherein the phenyl may be substituted with up to three substituents and wherein the naphthyl may be substituted with up to two substituents and the substituents are each independently selected from halogen, ($C_1$-$C_4$)alkyl, ($C_1$-$C_4$)alkoxy, nitro, trihalomethyl, ($C_1$-$C_4$)alkylthio and phenyl ; and acid salts, free bases and metal salt complexes thereof.

2. A compound of claim 1 wherein Het is pyrazinyl or 5-pyrimidinyl.

3. A compound of claim 1 or 2 wherein Ar is a phenyl group which is optionally substituted with up to two substituents each independently selected from halogen, ($C_1$-$C_4$)alkyl, ($C_1$-$C_4$)alkoxy, nitro, trihalomethyl, ($C_1$-$C_4$)alkylthio and phenyl.

4. A compound of any preceding claim wherein R is a ($C_1$-$C_6$)alkyl, halo($C_1$-$C_6$)alkyl having up to four halogen atoms or phenyl, wherein the phenyl may be substituted with up to two substituents each independently selected from halogen and trihalomethyl.

5. A compound of Claim 4 wherein Ar is a phenyl group substituted with up to two substituents each independently selected from halogen, trifluoromethyl, and phenyl.

6. A compound which is
2-(4-chlorophenyl)-2-cyano-1-pyrazinylhexane,
2-(4-chlorophenyl)-2-cyano-1-(5-pyrimidinyl)hexane,
2-(4-chlorophenyl)-2-cyano-1-pyrazinyl-(6,6,6-trifluoro)hexane,
2-cyano-2-(4-phenylphenyl)-1-pyrazinylhexane,
2-cyano-2-phenyl-4-trifluoromethylphenyl-1-pyrazinylbutane,
2-cyano-2-phenyl-4-trifluoromethylphenyl-1-(5-pyrimidinyl)butane or
2-cyano-2,4-di-(4-chlorophenyl)-1-pyrazinylbutane.

7. A fungicidal composition comprising a compound according to any preceding claim and an agronomically acceptable carrier or diluent.

8. A method for controlling a phytopathogenic fungus comprising applying to the fungus or its habitat, for example a plant, a plant habitat, plant seed or soil, a fungicidally-effective amount of a compound of claim 1 optionally in a composition according to Claim 7.

**Claims for the contracting State : ES**

1. The use of one or more compounds of the formula

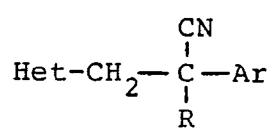

wherein Het is pyrazinyl, pyrimidinyl or pyridazinyl ;

R is hydrogen, ($C_1$-$C_8$)alkyl, ($C_3$-$C_6$)cycloalkyl, ($C_3$-$C_6$)cycloalkyl($C_1$-$C_4$)alkyl, halo($C_1$-$C_8$)alkyl, ($C_3$-$C_6$)alkenyl, halo($C_3$-$C_5$)alkenyl, ($C_3$-$C_6$)alkynyl, ($C_1$-$C_5$)alkoxy, ($C_2$-$C_5$)alkenoxy, ($C_2$-$C_5$)alkynoxy, substituted or unsubstituted phenyl, substituted or unsubstituted naphthyl, substituted or unsubstituted phenoxy, S or U phenyl($C_1$-$C_4$)alkyl, or S or U phenoxy ($C_2$-$C_4$)alkyl, wherein when R is a phenyl, phenoxy, naphthyl, phenylalkyl or phenoxyalkyl, the phenyl portion may optionally be substituted with up to two substituents each independently selected from halogen, nitro, trihalomethyl, cyano, ($C_1$-$C_4$)alkyl, ($C_1$-$C_4$)alkoxy, alkoxyalkyl having up to a total of four carbon atoms, ($C_1$-$C_4$)alkylthio, ($C_1$-$C_4$)alkylsulfinyl and ($C_1$-$C_4$)alkylsulfonyl groups ; and

Ar is a substituted or unsubstituted phenyl or naphthyl group, wherein the phenyl may be substituted with up to three substituents and wherein the naphthyl may be substituted with up to two substituents and the substituents are each independently selected from halogen, ($C_1$-$C_4$)alkyl, ($C_1$-$C_4$)alkoxy, nitro, trihalomethyl, ($C_1$-$C_4$)alkylthio and phenyl ; and acid salts, free bases and metal salt complexes thereof, together with agronomically acceptable diluent or carrier to make a fungicidal composition.

2. The use according to Claim 1 of a compound of formula I wherein Het is pyrazinyl or 5-pyrimidinyl.

3. The use according to Claim 1 or 2 of a compound of formula I wherein Ar is a phenyl group which is optionally substituted with up to two substituents each independently selected from halogen, ($C_1$-$C_4$)alkyl, ($C_1$-$C_4$)alkoxy, nitro, trihalomethyl, ($C_1$-$C_4$)alkylthio and phenyl.

4. The use according to any preceding claim of a compound of formula I wherein R is a ($C_1$-$C_6$)alkyl, halo($C_1$-$C_6$)alkyl having up to four halogen atoms or phenyl, wherein the phenyl may be substituted with up to two

substituents each independently selected from halogen and trihalomethyl.

5. The use according to claim 4 of a compound of formula I wherein Ar is a phenyl group substituted with up to two substituents each independently selected from halogen, trifluoromethyl, and phenyl.

6. The use according to Claim 1 of a compound which is
2-(4-chlorophenyl)-2-cyano-1-pyrazinylhexane,
2-(4-chlorophenyl)-2-cyano-1-(5-pyrimidinyl)hexane,
2-(4-chlorophenyl)-2-cyano-1-pyrazinyl-(6,6,6-trifluoro)hexane,
2-cyano-2-(4-phenylphenyl)-1-pyrazinylhexane,
2-cyano-2-phenyl-4-trifluoromethylphenyl-1-pyrazinylbutane,
2-cyano-2-phenyl-4-trifluoromethylphenyl-1-(5-pyrimidinyl)butane or
2-cyano-2,4-di-(4-chlorophenyl)-1-pyrazinylbutane.

7. The use of a compound, salt, free base or metal salt complex as defined in any one of Claims 1 to 6, optionally in a composition also containing agronomically acceptable diluent or carrier for controlling a phytopathogenic fungus by contacting said fungus, of a locus susceptible to attack by such a fungus, with an effective amount of said compound, salt, free base or metal salt complex.

8. A mechanical process for improving the commercial value and/or profitability of vendible crops from plants whose growth is affected or likely to be affected by a phytopathogenic fungus comrising (1) charging to a container, fumigation device or mechanical dissemination device a fungicaidal compound, salt, free base or metal salt complex as defined in any of claims 1 to 6, optionally in a mixture with agronomically acceptable diluent or carrier, (2) using the container, fumigator or mechanical dissemination device to apply the insecticidal compound or salt, in the form of granules, dust, smoke, vapour or surfactant-containing liquid preparation to growing plants, to plant seeds or to a growth medium where the plants are growing or are to be grown, or to the fungus itself, (3) controlling the dose of the active ingredient during this application step so that the rate of application of active fungicidal compound is sufficient to combat the fungus but is insufficient to cause an unacceptably adverse effect on the crop plants growing or to be grown in the treated area.

9. A method for controlling a phytopathogenic fungus comprising applying to the fungus or its habitat, for example a plant, a plant habitat, plant seed or soil, a fungicidally-effective amount of a compound of claim 1 optionally in a composition according to Claim 7.

## Ansprüche

**Patentansprüche für die Vertragsstaaten : AT, BE, CH, DE, FR, GB, GR, IT, LI, LU, NL, SE**

1. Verbindungen der Formel

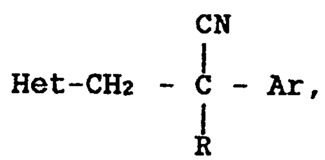

wobei Het die Bedeutung Pyrazinyl, Pyrimidinyl oder Pyridazinyl hat ;

R bedeutet :

Wasserstoff, $(C_1\text{-}C_8)$Alkyl, $(C_3\text{-}C_6)$Cycloalkyl, $(C_3\text{-}C_6)$Cycloalkyl$(C_1\text{-}C_4)$alkyl, Halo$(C_1\text{-}C_8)$alkyl, $(C_3\text{-}C_6)$Alkenyl, Halo$(C_3\text{-}C_5)$alkenyl, $(C_3\text{-}C_8)$Alkynyl, $(C_1\text{-}C_5)$Alkoxy, $(C_2\text{-}C_5)$Alkenoxy, $(C_2\text{-}C_5)$Alkynoxy, substituiertes oder unsubstituiertes Phenyl, substituiertes oder unsubstituiertes Naphthyl, substituiertes oder unsubstituiertes Phenoxy, substituiertes oder unsubstituiertes Phenyl-$(C_1\text{-}C_4)$alkyl, oder substituiertes oder unsubstituiertes Phenoxy $(C_2\text{-}C_4)$alkyl, wobei dann, wenn R die Bedeutung Phenyl, Phenoxy, Naphthyl, Phenylalkyl oder Phenoxyalkyl hat, der Phenylteil wahlweise substituiert sein kann mit bis zu zwei Substituenten, die jeweils unabhängig ausgewählt sind aus Halogen, Nitro, Trihalomethyl, Cyano, $(C_1\text{-}C_4)$Alkyl, $(C_1\text{-}C_4)$Alkoxy, Alkoxyalkyl mit bis zu insgesamt vier Kohlenstoffatomen, $(C_1\text{-}C_4)$Alkylthio-, $(C_1\text{-}C_4)$Alkylsulfinyl- und $(C_1\text{-}C_4)$Alkylsufonyl-Gruppen ; und

Ar eine substituierte oder unsubstituierte Phenyl- oder Naphthylgruppe bedeutet, wobei das Phenyl mit bis zu drei Substituenten substituiert sein kann und wobei das Naphthyl mit bis zu zwei Substituenten substituiert sein kann, und die Substituenten jeweils unabhängig ausgewählt sind aus Halogen, $(C_1\text{-}C_4)$Alkyl, $(C_1\text{-}C_4)$Alkoxy, Nitro, Trihalomethyl, $(C_1\text{-}C_4)$Alkylthio und Phenyl ; und Säuresalze, freie Basen und Metallsalzkomplexe davon.

2. Verbindung nach Anspruch 1, wobei Het die Bedeutung Pyrazinyl oder 5-Pyrimidinyl hat.

3. Verbindung nach Anspruch 1 oder 2, wobei Ar eine Phenylgruppe bedeutet, die wahlweise mit bis zu zwei Substituenten substituiert ist, die jeweils unabhängig ausgewählt sind aus Halogen, $(C_1$-$C_4)$Alkyl, $(C_1$-$C_4)$Alkoxy, Nitro, Trihalomethyl, $(C_1$-$C_4)$Alkylthio und Phenyl.

4. Verbindung nach einem Beliebigen vorhergehenden Anspruch, wobei R die Bedeutung $(C_1$-$C_6)$Alkyl, Halo$(C_1$-$C_6)$alkyl mit bis zu vier Halogenatomen oder Phenyl hat, wobei das Phenyl mit bis zu zwei Substituenten substituiert sein kann, die jeweils unabhängig ausgewählt sind aus Halogen und Trihalomethyl.

5. Verbindung nach Anspruch 4, wobei Ar eine mit bis zu zwei Substituenten substituierte Phenylgruppe bedeutet, wobei die Substituenten jeweils unabhängig ausgewählt sind aus Halogen, Trifluormethyl und Phenyl.

6. Verbindung, welche ist
2-(4-Chlorphenyl)-2-cyano-1-pyrazinylhexan,
2-(4-Chlorphenyl)-2-cyano-1-(5-pyrimidinyl)hexan,
2-(4-Chlorphenyl)-2-cyano-1-pyrazinyl-(6,6,6-trifluor)-hexan,
2-Cyano-2-(4-phenylphenyl)-1-pyrazinylhexan,
2-Cyano-2-phenyl-4-trifluormethylphenyl-1-pyrazinylbutan,
2-Cyano-2-phenyl-4-trifluormethylphenyl-1-(5-pyrimidinyl)butan oder
2-Cyano-2,4-di-(4-chlorphenyl)-1-pyrazinylbutan.

7. Fungizide Zusammensetzung, enthaltend eine Verbindung nach einem beliebigen vorhergehenden Anspruch und einen landwirtschaftlich vertretbaren Träger- oder Verdünnungsstoff.

8. Verfahren zum Kontrollieren eines phytopathogenen Pilzes, bei dem man dem Pilz oder seinem Habitat, z.B. einer Pflanze, einem Pflanzenhabitat, Pflanzensamen oder Boden eine fungizid wirksame Menge einer Verbindung nach Anspruch 1 zuführt, wahlweise in einer Zusammensetzung gemäß Anspruch 7.

**Patentansprüche für den Vertragsstaat : ES**

1. Verwendung von einer oder von mehreren Verbindungen der Formel

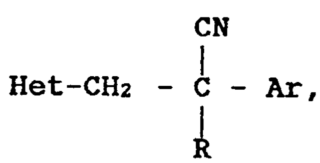

wobei Het die Bedeutung Pyrazinyl, Pyrimidinyl oder Pyridazinyl hat ;

R bedeutet :

Wasserstoff, $(C_1$-$C_8)$Alkyl, $(C_3$-$C_6)$Cycloalkyl, $(C_3$-$C_6)$Cycloalkyl$(C_1$-$C_4)$alkyl, Halo$(C_1$-$C_8)$alkyl, $(C_3$-$C_6$ ) Alkenyl, Halo$(C_3$-$C_5)$alkenyl, $(C_3$-$C_6)$Alkynyl, $(C_1$-$C_5)$Alkoxy, $(C_2$-$C_5)$Alkenoxy, $(C_2$-$C_5)$Alkynoxy, substituiertes oder unsubstituiertes Phenyl, substituiertes oder unsubstituiertes Naphthyl, substituiertes oder unsubstituiertes Phenoxy, substituiertes oder unsubstituiertes Phenyl $(C_1$-$C_4)$alkyl, oder substituiertes oder unsubstituiertes Phenoxy $(C_2$-$C_4)$alkyl, wobei dann, wenn R die Bedeutung Phenyl, Phenoxy, Naphthyl, Phenylalkyl oder Phenoxyalkyl hat, der Phenylteil wahlweise substituiert sein kann mit bis zu zwei Substituenten, die jeweils unabhängig ausgewählt sind aus Halogen, Nitro, Trihalomethyl, Cyano, $(C_1$-$C_4)$Alkyl, $(C_1$-$C_4)$Alkoxy, Alkoxyalkyl mit bis zu insgesamt vier Kohlenstoffatomen, $(C_1$-$C_4)$Alkylthio-, $(C_1$-$C_4)$Alkylsulfinyl- und $(C_1$-$C_4)$Alkylsufonyl-Gruppen ; und

Ar eine substituierte oder unsubstituierte Phenyl- oder Naphthylgruppe bedeutet, wobei das Phenyl mit bis zu drei Substituenten substituiert sein kann und wobei das Naphthyl mit bis zu zwei Substituenten substituiert sein kann, und die Substituenten jeweils unabhängig ausgewählt sind aus Halogen, $(C_1$-$C_4)$Alkyl, $(C_1$-$C_4)$Alkoxy, Nitro, Trihalomethyl, $(C_1$-$C_4)$Alkylthio und Phenyl ; und Säuresalze, freie Basen und Metallsalzkomplexe davon, zusammen mit landwirtschaftlich vertretbarem Verdünnungs- oder Trägerstoff zur Herstellung einer fungiziden Zusammensetzung.

2. Verwendung nach Anspruch 1 einer Verbindung der Formel I, wobei Het die Bedeutung Pyrazinyl oder 5-Pyrimidinyl hat.

3. Verwendung nach Anspruch 1 oder 2 einer Verbindung der Formel I, wobei Ar eine Phenylgruppe bedeutet, die wahlweise mit bis zu zwei Substituenten substituiert ist, die jeweils unabhängig ausgewählt sind aus Halogen, $(C_1$-$C_4)$Alkyl, $(C_1$-$C_4)$Alkoxy, Nitro, Trihalomethyl, $(C_1$-$C_4)$Alkylthio und Phenyl.

4. Verwendung nach einem beliebigen vorhergehenden Anspruch einer Verbindung der Formel I, wobei

R die Bedeutung ($C_1$-$C_6$)Alkyl, Halo($C_1$-$C_6$)alkyl mit bis zu vier Halogenatomen oder Phenyl hat, wobei das Phenyl mit bis zu zwei Substituenten substituiert sein kann, die jeweils unabhängig ausgewählt sind aus Halogen und Trihalomethyl.

5. Verwendung nach Anspruch 4 einer Verbindung der Formel I, wobei Ar eine mit bis zu zwei Substituenten substituierte Phenylgruppe bedeutet, wobei die jeweils Substituenten unabhängig ausgewählt sind aus Halogen, Trifluormethyl und Phenyl.

6. Verwendung gemäß Anspruch 1 einer Verbindung, welche ist
2-(4-Chlorphenyl)-2-cyano-1-pyrazinylhexan,
2-(4-Chlorphenyl)-2-cyano-1-(5-pyrimidinyl)hexan,
2-(4-Chlorphenyl)-2-cyano-1-pyrazinyl-(6,6,6-trifluor)-hexan,
2-Cyano-2-(4-phenylphenyl)-1-pyrazinylhexan,
2-Cyano-2-phenyl-4-trifluormethylphenyl-1-pyrazinylbutan,
2-Cyano-2-phenyl-4-trifluormethylphenyl-1-(5-pyrimidinyl)butan oder
2-Cyano-2,4-di-(4-chlorphenyl)-1-pyrazinylbutan.

7. Verwendung einer Verbindung, eines Salzes, einer freien Base oder eines Metallsalzkomplexes wie in einem beliebigen der Ansprüche 1 bis 6 definiert, wahlweise in einer Zusammensetzung weiter enthaltend landwirtschaftlich vertretbaren Verdünnungs- oder Trägerstoff zum Kontrollieren eines phytopathogenen Pilzes durch Kontaktieren dieses Pilzes, eines dem Angriff durch eines solchen Pilzes zugänglichen Lokus mit einer wirksamen Menge der Verbindung, des Salzes, der freien Base oder des Metallsalzkomplexes.

8. Mechanisches Verfahren zum Verbessern des Handelswertes und/oder der Rentabilität verkaufbarer Ernten aus Pflanzen, deren Wachstum von einem phytopathogenen Pilz beeinflußt wird oder möglicherweise beeinflußt wird, bei dem man

(1) einen Behälter, einer Vernebelungseinrichtung oder einem mechanischen Ausbreiter eine fungizide Verbindung, Salz, freie Base oder Metallsalzkomplex gemäß einem beliebigen der Ansprüche 1 bis 6 zuführt, wahlweise in einer Mischung mit landwirtschaftlich vertretbarem Verdünnungs- oder Trägerstoff,
(2) den Behälter, Vernebeler oder mechanischen Ausbreiter dazu verwendet, die fungizide Verbindung oder das Salz in Form von Granulat, Staub, Rauch, Dampf oder einer tensidhaltigen Flüssigkeitszubereitung wachsenden Pflanzen, Pflanzensamen oder einem Wachstumsmedium zu applizieren, wo die Pflanzen wachsen oder gezogen werden, oder dem Pilz selbst,
(3) die Dosis des Wirkstoffs während des Applizierungsschrittes so steuert, daß die Applizierungsrate der aktiven fungiziden Verbindung zur Bekämpfung des Pilzes ausreicht, jedoch nicht ausreicht zur Erzeugung einer nicht hinnehmbaren nachteiligen Auswirkung auf die in dem behandelnden Gebiet wachsenden oder zu ziehenden Erntepflanzen.

9. Verfahren zum Kontrollieren eines phytopathogenen Pilzes, bei dem man dem Pilz oder seinem Habitat, z.B. einer Pflanze, einem Pflanzenhabitat, Pflanzensamen oder Boden eine fungizid wirksame Menge einer Verbindung nach Anspruch 1 zuführt, wahlweise in einer Zusammensetzung gemäß Anspruch 7.

## Revendications

### Revendications pour les Etats contractants : AT, BE, CH, DE, FR, GB, Gr, IT, LI, LU, NL, SE

1. Composés de formule

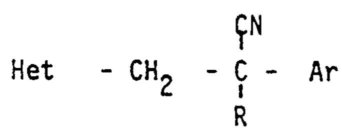

dans laquelle Het représente un groupe pyrazinyle, pyrimidinyle ou pyridazinyle ;

R représente un atome d'hydrogène,un groupe alkyle en $C_1$ à $C_8$, cycloalkyle en $C_3$ à $C_6$, cycloalkyl (en $C_3$ à $C_6$)-alkyle en $C_1$ à $C_4$, halogéno-alkyle en $C_1$ à $C_8$, alcényle en $C_3$ à $C_6$, halogéno-alcényle en $C_3$ à $C_5$, alcynyle en $C_3$ à $C_6$, alcoxy en $C_1$ à $C_5$, alcénoxy en $C_2$ à $C_5$, alcynoxy en $C_2$ à $C_5$, phényle substitué ou non substitué, naphtyle substitué ou non substitué, phénoxy substitué ou non substitué, phényl alkyle (en $C_1$ à $C_4$) substitué ou non substitué, ou phénoxy alkyle (en $C_2$ à $C_4$) susbtitué ou non substitué et, lorsque R représente un groupe phényle, phénoxy, naphtyle, phénylalkyle ou phénoxy alkyle, la partie phényle peut porter jusqu'à deux substituants au maximum, choisis chacun, indépendamment, parmi un atome

d'halogène, un groupe nitro, trihalogénométhyle, cyano, alkyle en $C_1$ à $C_4$, alcoxy en $C_1$ à $C_4$, alcoxyalkyle ayant jusqu'à un total de quatre atomes de carbone au maxixmum, alkylihio en $C_1$ à $C_4$, alkylsulfinyle en $C_1$ à $C_4$ et alkylsulfonyle en $C_1$ à $C_4$ ; et

Ar représente un groupe phényle ou naphtyle, substitué ou non substitué, dans lequel le groupe phényle peut comporter jusqu'à trois substituants et le groupe naphtyle peut comporter jusqu'à deux substituants, et les substituants sont choisis chacun, indépendamment, parmi un atome d'halogène, un groupe alkyle en $C_1$ à $C_4$, alcoxy en $C_1$ à $C_4$, nitro, trihalogénométhyle, alkylthio en $C_1$ à $C_4$ et phényle ; et leurs sels d'acides, leurs bases libres et leurs complexes avec des sels de métaux.

2. Composé selon la revendication 1, dans lequel Het représente un groupe pyrazinyle ou 5-pyrimidinyle.

3. Composé selon la revendication 1 ou 2, dans lequel Ar représente un groupe phényle comportant éventuellement jusqu'à deux substituants choisis chacun, indépendamment, parmi un atome d'halogène, un groupe alkyle en $C_1$ à $C_4$, alcoxy en $C_1$ à $C_4$, nitro, trihalogénométhyle, alkylthio en $C_1$ à $C_4$ et phenyle.

4. Composé selon l'une quelconque des revendications précédentes, dans lequel R représente un groupe alkyle en $C_1$ à $C_6$, halogéno-alkyle en $C_1$ à $C_6$ ayant jusqu'à quatre atomes d'halogène, ou phényle, le groupe phényle pouvant porter jusqu'à deux substituants choisis chacun, indépendamment, parmi un atome d'halogène et un groupe trihalogénométhyle.

5. Composé selon la revendication 4, dans lequel Ar représente un groupe phényle portant jusqu'à deux substituants choisis chacun indépendamment parmi un groupe d'halogène, un groupe trifluorométhyle et phényle.

6. Composé qui est
le 2-(4-chlorophényl)-2-cyano-1-pyrazinylhexane,
le 2-(4-chlorophényl)-2-cyano-1-(5-pyrimidinyl)hexane,
le 2-(4-chlorophényl)-2-cyano-1-pyrazinyl-(6,6,6-trifluoro)hexane,
le 2-cyano-2-(4-phénylphényl)-1-pyrazinylhexane,
le 2-cyano-2-phényl-4-trifluorométhylphényl-1-pyrazinylbutane,
le 2-cyano-2-phényl-4-trifluorométhylphényl-1-(5-pyrimidinyl)butane, ou
le 2-cyano-2,4-di-(4-chlorophényl)-1-pyrazinylbutane.

7. Composition fongicide comprenant un composé selon l'une quelconque des revendications précédentes et un support, véhicule, excipient ou diluant agronomiquement acceptable.

8. Procédé pour maîtriser un champignon phytopathogène, comprenant l'application au champignon ou à son habitat, par exemple une plante, un habitat de plante, une graine de semence de plante ou le sol, d'une quantité, efficace du point de vue fongicide, d'un composé selon la revendication 1, éventuellement dans une composition selon la revendication 7.

**Revendications pour l'Etat contractant : ES**

1. Utilisation d'un ou plusieurs composés de formule :

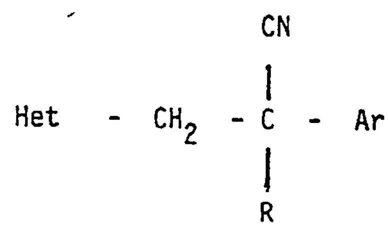

dans laquelle Het représente un groupe pyrazinyle, pyrimidinyle ou pyridazinyle ;

R représente un atome d'hydrogène,un groupe alkyle en $C_1$ à $C_8$, cycloalkyle en $C_3$ à $C_6$, cyclo-alkyl (en $C_3$ à $C_6$)-alkyle (en $C_1$ à $C_4$), halogéno-alkyle en $C_1$ à $C_8$, alcényle en $C_3$ à $C_6$, halogéno-alcényle en $C_3$ à $C_5$, alcycnyle en $C_3$ à $C_6$, alcoxy en $C_1$ à $C_5$, alcénoxy en $C_2$ à $C_5$, alcynoxy en $C_2$ à $C_5$, phényle substitué ou non substitué, naphtyle substitué ou non substitué, phénoxy substitué ou non substitué, phényl alkyle (en $C_1$ à $C_4$) substitué ou non substitué ou phénoxy-alkyle (en $C_2$ à $C_4$) substitué ou non substitué, et lorsque R représente un groupe phényle, phénoxy, napthyle, phénylalkyle ou phénoxyalkyle, la partie phényle peut éventuellement porter comme substituants jusqu'à deux substituants, chacun choisi indépendamment parmi un atome d'halogène, un groupe nitro, trihalogénométhyle, cyano, alkyle en $C_1$ à $C_4$, alcoxy en $C_1$ à $C_4$, alcoxyalkyle ayant jusqu'à un nombre total maximal de quatre atomes de carbone, alkylthio en $C_1$ à $C_4$, alkylsulfinyle en $C_1$ à $C_4$ et alkylsulfonyle en $C_1$ à $C_4$ ; et

Ar représente un groupe phényle ou naphtyle substitués ou non substitués, le groupe phényle pouvant porter jusqu'à un maximum de trois substituants et le groupe napthyle pouvant porter jusqu'à un maximum de deux substituants, et les substituants sont choisis, chacun indépendemment, parmi un atome d'halogène, un groupe alkyle en $C_1$ à $C_4$, alcoxy en $C_1$ et $C_4$ ; nitro, trihalogénométhyle, alkylthio en $C_1$ à $C_4$ et phényle ; et son ou leurs sels d'acide, bases libres et complexes avec des sels de métaux, avec un diluant ou un excipient, support ou véhicule agronomiquement acceptables, pour produire une composition fongicide.

2. Utilisation selon la revendication 1 d'un composé de formule I dans laquelle Het représente un groupe pyrazinyle ou 5-pyrimidinyle.

3. Utilisation, selon la revendication 1 ou 2, d'un composé de formule I dans laquelle Ar représente un groupe phényle portant éventuellement jusqu'à un maximum de deux substituants choisis chacun, indépendamment, parmi un atome d'halogène, un groupe alkyle en $C_1$ à $C_4$, alcoxy en $C_1$ à $C_4$, nitro, trihalogénométhyle, alkylthio en $C_1$ à $C_4$ et phényle.

4. Utilisation, selon l'une quelconque des revendications précédentes, d'un composé de formule I dans laquelle R représente un groupe alkyle en $C_1$ à $C_6$, halogéno-alkyle en $C_1$ à $C_6$ ayant jusqu'à un maximum de quatre atomes d'halogène ou phényle, le groupe phényle pouvant porter jusqu'à un maximum de deux substituants, choisis chacun indépendamment parmi un atome d'halogène et un groupe trihalogénométhyle.

5. Utilisation, selon la revendication 4, d'un composé de formule I, dans laquelle Ar est un groupe phényle portant jusqu'à un maximum de deux substituants choisis chacun, indépendamment, parmi un atome d'halogène, un groupe trifluorométhyle et phényle.

6. Utilisation, selon la revendication 1, d'un composé qui est
le 2-(4-chlorophényl)-2-cyano-1-pyrazinylhexane,
le 2-(4-chlorophényl)-2-cyano-1-(5-pyrimidinyl)hexane,
le 2-(4-chlorophényl)-2-cyano-1-pyrazinyl-(6,6,6-trifluoro)hexane,
le 2-cyano-2-(4-phénylphényl)-1-pyrazinylhexane,
le 2-cyano-2-phényl-4-trifluorométhylphényl-1-pyrazinylbutane,
le 2-cyano-2-phényl-4-trifluorométhylphényl-1-(5-pyrimidinyl)butane ou
le 2-cyano-2,4-di-(4-chlorophényl)-1-pyrazinylbutane.

7. Utilisation d'un composé, d'un sel, d'une base libre ou d'un complexe de sel de métal tel que défini dans l'une quelcopnque des revendications 1 à 6, éventuellement dans une composition contenant aussi un diluant, excipient, véhicule ou support, pour maîtriser un champignon phytopathogène par la mise en contact dudit champignon, d'un lieu susceptible de subir une attaque par un tel champignon, avec une quantité efficace dudit composé, sel, base libre ou complexe de sel de métal.

8. Procédé mécanique pour améliorer la valeur commerciale et/ou la rentabilité de récoltes vendables ou marchandes de plantes dont la croissance est affectée, ou risque d'être affectée, par un champignon phytopathogène, ce procédé comprenant (1) l'introduction dans un récipient, un dispositif de fumigation ou un dispositif de dissémination mécanique, d'un composé fongicide, d'un sel, d'une base libre, ou d'un complexe de sel de métal, tel que défini dans l'une quelconque des revendications 1 à 6, éventuellement en mélange avec un diluant, ou unsupport, véhicule ou excipient agronomiquement acceptable ; (2) l'utilisation du récipient, fumigateur ou dispositif de dissémination mécanique, pour appliquer le composé ou sel insecticide, sous forme de granules, de poussière fine, de fumée, de vapeur ou d'une préparation liquide contenant du surfactif, à des plantes en cours de croissance, à des semences de plantes ou à un milieu de croissance dans lequel les plantes sont en cours de croissance ou doivent être cultivées, ou bien au champignon lui-même, (3) le réglage de la dose de l'ingrédient actif durant cette étape d'application de sorte que le taux d'application du composé fongicide actif soit suffisant pour combattre le champignon, mais ne suffise pas à provoquer un effet adverse inacceptable sur les plantes cultivées, en cours de croissance ou devant être cultivées dans la zone traitée.

9. Procédé pour maîtriser un champignon phytopathogène, ce procédé comprenant l'application au champignon ou à son habitat, par exemple une plante, un habitat ou biotope de plante, une graine de semence de plante ou un sol, d'une quantité, efficace du point de vue fongicide, d'un composé selon la revendication 1, éventuellement dans une composition selon la revendication 7.